# EUROPEAN PATENT SPECIFICATION

(11) **EP 0 787 302 B1**
(45) Date of publication and mention of the grant of the patent: **11.02.2009**
(21) Application number: 95935750.0
(22) Date of filing: 23.10.1995
(51) Int. Cl.: G01N 33/74, G01N 33/53

(54) **METHOD FOR DETERMINING TERM OF PREGNANCY**
VERFAHREN ZUR BESTIMMUNG DES SCHWANGERSCHAFTSSTADIUMS
PROCEDE PERMETTANT DE DETERMINER LE TERME DE LA GROSSESSE

(30) Priority: 21.10.1994 AU PM897994
(43) Date of publication of application: 06.08.1997
(73) Proprietor: UNIVERSITY OF NEWCASTLE, Newcastle, NSW 2308 (AU)
(72) Inventor: SMITH, Roger, Newcastle, NSW 2300 (AU)
(74) Representative: Chapman, Paul William
(86) International application number: PCT/AU1995/000710
(87) International publication number: WO 1996/012967

(56) References cited:
- WO-A-93/24836
- WO-A-93/24838
- RILEY ET AL: "The Localistion and Distribution of Corticotropin-Releasing Hormone in the Human Placenta and Fetal Membranes throughout Gestation" JOURNAL OF CLINICAL ENDOCRINOLOGY AND METABOLISM, vol. 72, no. 5, 1991, pages 1001-1006, XP002109972 US
- CAMPELL ET AL: "Plasma Corticotropin-Releasing Hormone Concentrations During Pregnancy and Parturition" JOURNAL OF CLINICAL ENDOCRINOLOGY AND METHOD, vol. 64, no. 5, 1987, pages 1054-1058, XP002109973 us
- BRITISH JOURNAL OF OBSTETRICS AND GYNAECOLOGY, October 1988, Vol. 95, WOLFE C.D.A. et al., "Plasma Corticotrophin-Releasing Factor (CRF) in Abnormal Pregnancy", pages 1003-1006. XP000671864
- CHIEN ET AL: 'The diagnostic accuracy of cervico-vaginal foetal fibronectin in predicting preterm delivery: an overview' BR.J.OBSTET GYNAECOL. vol. 104, 1997, pages 436 - 444

## Description

### TECHNICAL FIELD

The present invention relates to a method of determining an anticipated time for parturition in a pregnant human subject and in particular relates to a method for the early detection of premature or post-mature delivery by the subject.

### BACKGROUND ART

Parturition is a transitional phase occupying the final three weeks of gestation in humans, characterised by a series of structural and functional changes in the myometrium, cervix and foetal membranes which culminate in the onset of labour and delivery of the baby. It has long been assumed that parturition is initiated by a hormonal event in late pregnancy, but no such event has yet been identified in human pregnancy⁽¹⁾. Not all women undergo full term pregnancy and any given pregnancy may result in premature or post-mature delivery. A premature delivery may result in the birth of an underweight and immature baby that will require intensive medical management before the baby may return home with the mother. In severe cases, premature delivery may lead to brain damage and other severe complications or result in the death of the baby. Alternatively, a pregnancy may result in a post-mature delivery which may also have severe consequences to the mother and baby. It is therefore desirable to have a method of early detection or prediction that a given pregnancy may result in premature or post-mature delivery.

### DESCRIPTION OF THE INVENTION

During the study of levels of corticotrophin-releasing hormone (CRH) and CRH-binding protein (CRH-BP) in pregnant humans, the present inventor has developed a method of determining an anticipated time for parturition in humans.

The present inventor has shown that the timing of human parturition is influenced from an early stage in pregnancy by a longitudinal maturation process in the foetal-placental unit. Measurement of maternal CRH levels has been shown by the inventor to provide a marker for this process.

Accordingly, in a first aspect the present invention consists in a method for detection or prediction of premature delivery in a pregnant human subject, the method comprising:
measuring during the first or second trimester of that pregnancy the maternal level of corticotrophin-releasing hormone (CRH);
comparing the level so obtained with the normal level of CRH, found in humans having a full term pregnancy and at the same stage of gestation as the pregnant human subject; and
determining that the delivery will occur sooner than full term if the level of CRH is elevated relative to the normal level of CRH.

In a preferred embodiment the invention provides a method for determining whether a pregnant human patient experiencing preterm uterine contractions is at risk of delivering prematurely.

In a second aspect, the present invention consists in a method of early detection or prediction of post-mature delivery in a pregnant human subject comprising:
measuring during the first or second trimester of that pregnancy the maternal level of corticotrophin-releasing hormone (CRH); comparing the level so obtained with the normal level of CRH found in humans having a full term pregnancy and at the same stage of gestation as the pregnant human subject; and
determining that the delivery will occur later than full term if the level of CRH is depressed relative to the normal level of CRH.

Current practice when pregnant women present in apparent preterm labour is to commence treatment with salbutamol to try to stop the contractions. Recently, a group of researchers (22) have demonstrated that transdermal nitric oxide donating agents may also provide effective treatment, permitting the pregnancy to progress to full term.

The present inventor has shown in a randomised comparison of these two methods of treatment that some patients responded poorly to the administered therapy and delivered prematurely inspite of the therapy. In cases where this happened, the patient showed significantly elevated CRH levels, which indicates that labour was imminent.

A significant problem for premature babies is lung immaturity. Foetal lungs can be matured by the administration of β methasone. However, if delivery is likely the β methasone needs to be administered quickly in order to permit lung maturation to occur prior to delivery.

A method of determining that delivery is imminent and that consequently tocolytic therapy may not succeed can improve patient care by facilitating clinical decision making with regard to administration of agents such as steroids to mature foetal lungs. This treatment can be undertaken as a priority followed by a decision regarding persevering with tocolytic therapy.

The levels of CRH can be measured in blood taken from the patient. Typically the levels are measured in the patient's plasma or serum.

It will be understood that the levels of CRH can be measured directly or indirectly by a variety of techniques available to the skilled addressee.

In a preferred embodiment of the first and second aspects, the measuring of CRH levels is by an immunological method. Preferably the immunological method is a radioimmunoassay.

If a premature or post-mature delivery in a pregnancy of a human subject is detected by the methods of the present invention, then it is possible to instigate medical management of the pregnancy so as to result in an improved outcome for the foetus. Such medical management could include the transdermal application of nitric oxide donating drugs, administration of salbutamol or the use of other drugs or treatments for the prevention of premature delivery. Other medical management may include transfer of the pregnant woman to specialist obstetric services and/or administration of steroid or other medication to promote foetal lung maturation.

The present invention would also be useful to confirm that any given pregnancy, at the time of measuring the level of CRH of the woman, should proceed to full term if the level of CRH measured was within normal range.

### BRIEF DESCRIPTION OF THE DRAWINGS

In order that the nature of the present invention may be more clearly understood, preferred forms thereof will be described with reference to the following examples and drawings:

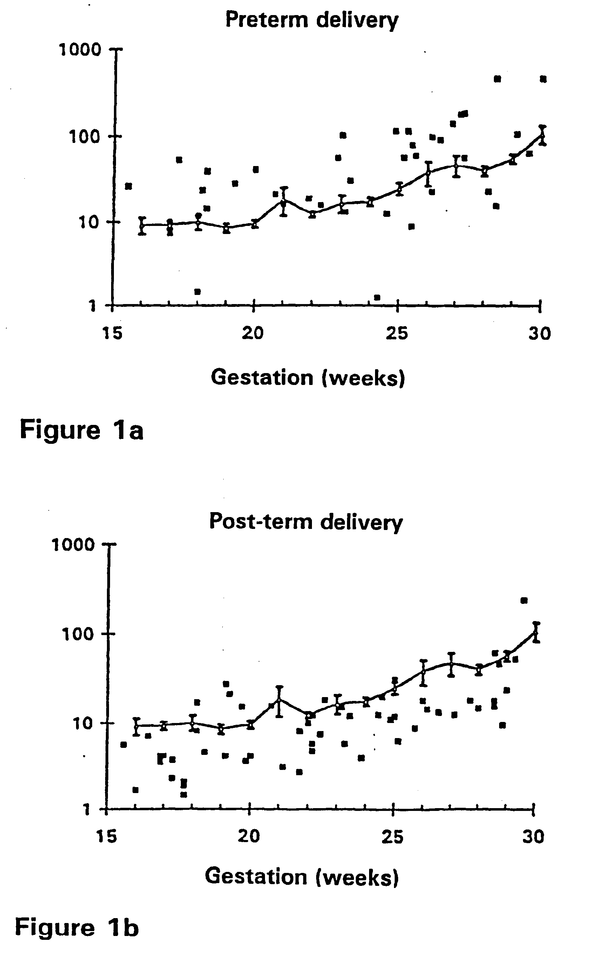

Figures 1 a and b shows maternal plasma CRH concentrations throughout midgestation in women whose pregnancies ended in spontaneous preterm labour (n=24), spontaneous term labour (n=308), or post-term delivery (n=29). In Figure 1a, mean (± s.e.m.) plasma CRH in women delivering at term (points with error bars) was compared with individual samples taken from women delivering preterm (■) or in b, postterm (■);

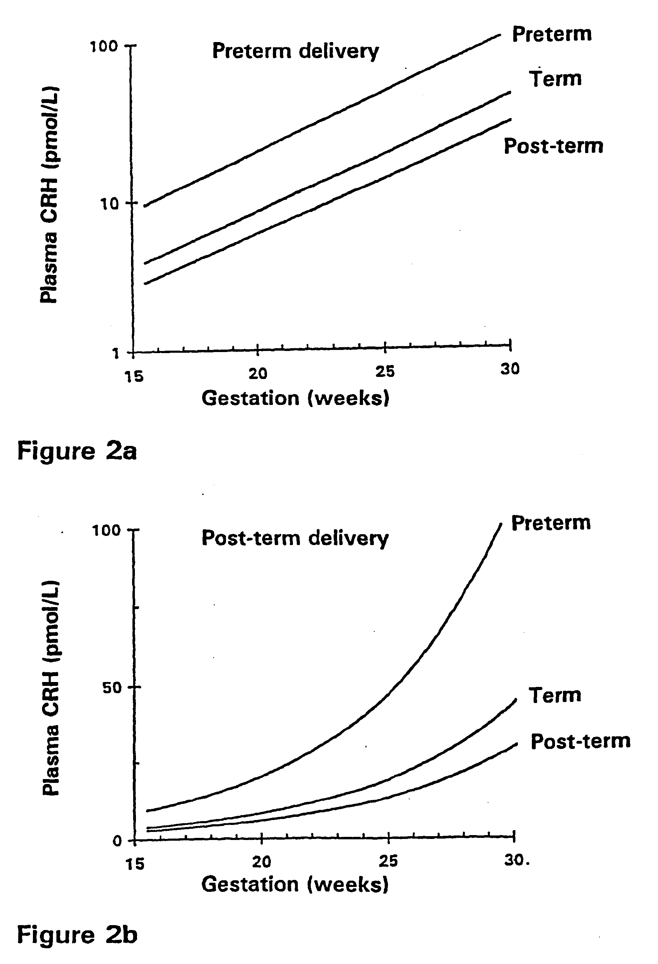

Figures 2 a and b provide a graphical representation of regression analysis of the levels shown in Figure 1. Figure 2a shows log linear regression curves fitted to plasma CRH results from the three subject groups. Figure 2b is a non-logarithmic representation of the three regression curves demonstrating the exponential increase of plasma CRH with advancing gestation; and

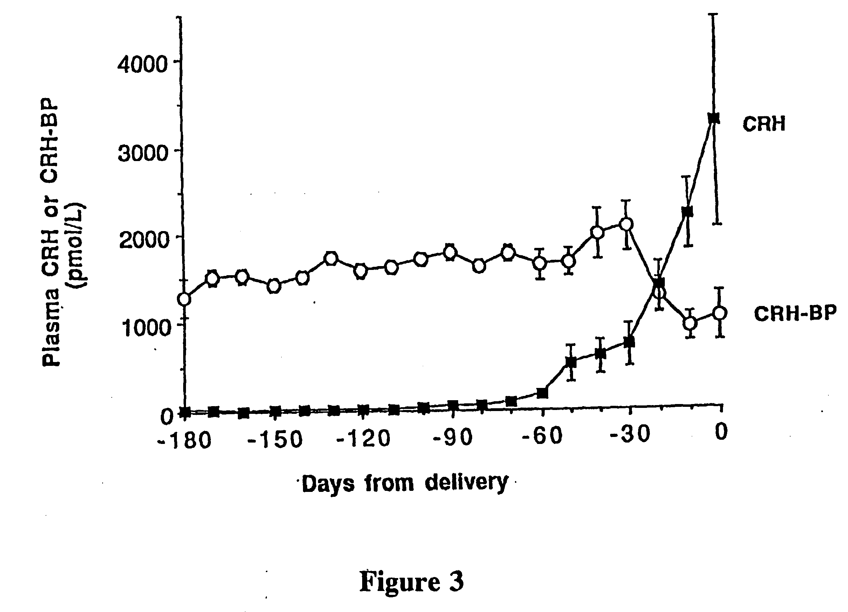

Figure 3 is a comparison of molar concentrations of CRH and CRH-BP in maternal plasma during the final 180 days of gestation in pregnancies ending in spontaneous term labour (37-42 weeks gestation).

### BEST METHOD OF CARRYING OUT THE INVENTION

Maternal CRH levels can be measured in plasma samples via a radioimmunoassay method that has been described previously(2).

### Example 1

The maternal plasma levels of CRH in pregnant women were measured throughout gestation by radioimmunoassay in extracted plasma as previously described⁽²⁾ and these levels were related to stage of gestation. The results are shown in Figures 1 and 2. Maternal plasma CRH concentrations were measured throughout mid-gestation in women whose pregnancies ended in spontaneous preterm labour (less than 37 weeks gestation, n = 24), full term labour (37-42 weeks, n = 308), or post-term delivery (induction of labour greater than 41.5 weeks or spontaneous labour greater than 42 weeks, n = 29). In Figures 2a and b the three curves are parallel and the CRH curve is shifted significantly to the left (by 6 weeks) in pregnancies delivering preterm (p 0.05), and significantly to the right (by 2 weeks) in pregnancies delivering post-term (p = 0.02). The median gestational age at delivery was 34 weeks in the preterm group, 40 weeks in the term group and 42 weeks in the post-term group. Each subject provided 1-4 samples (median = 2) which were collected at routine antenatal clinic visits at 4 to 6 week intervals. Gestational age was estimated by ultrasonography in 95% of subjects and by date of the last menstrual period in the remainder. Obstetric outcomes were obtained from a computerised database, and written case notes of all preterm deliveries were reviewed by two clinicians blinded to the CRH results. Women delivered by Caesarean section or after induction of labour are excluded from the analysis, except those already post-term at the time of delivery. Analysis of parallelism and shift of the regression curves was performed using the method of Kleinbaum *et al*⁽³⁾.

Comparison of the molar concentrations of CRH and CRH-BP in maternal plasma during the final 180 days of gestation in pregnancies ending in spontaneous term labour (37 to 42 weeks gestation) is shown in Figure 3. CRH-BP was measured by radioimmunoassay as previously described⁽⁴⁾. Each point represents the mean (± sem) of samples grouped by 10 day intervals calculated retrospectively from the day of delivery (mean of 59 samples at each point). This analysis includes all samples from the 308 women with spontaneous term delivery prospectively sampled for the present study, and additional samples (collected using identical methodology) from 124 women sampled throughout late pregnancy and parturition for a previously reported study⁽³⁾ CRH and CRH-BP were measured in duplicate aliquots. CRH and CRH-BP concentrations are significantly different (t-test, p less than 0.002) at all points except at the intersection of the 2 curves, 20 days before delivery.

### Example 2

In a randomised trial of tocolytic treatment of patients presenting in apparent preterm labour, maternal plasma CRH levels were measured by radioimmunossay in extracted plasma as previously described (2). The patients were treated with transdermal nitric oxide donating drugs or salbutamol. The results are shown in Table 1.

| | | | | | |
|---|---|---|---|---|---|
| Table 1 | | | | | |
| Patient No | Gestation (weeks) | Treatment | Length of Treatment | Basal CRH (pmol/L) | Gestation extended |
| 1 | 32 | NO(1)¹ | 12 hrs | 93.53 | 4 days |
| 2 | 33 | NO(1) | 12 hrs | 7.40 | 8 weeks |
| 3 | 32 | NO(1) | 12 hrs | 16.76 | 10 weeks |
| 4 | 30 | NO(2) Sal | Failed 15 hrs | 8.79 | 11 weeks |
| 5 | 29 | Sal | 12 hrs | 8.34 | 10 weeks |
| 6 | 33 | NO(2) | 48 hrs | 8.80 | 9 weeks |
| 7 | 26 | Sal | 12 hrs | 3.19 | 15 weeks |
| 8 | 26 | Sal | 12 hrs | 4.88 | 14 weeks |
| 9 | 28 | Sal | 12 hrs | 4.72 | 12 weeks |
| 10 | 29 | Sal | 12 hrs | 28.45 | 1 day |
| 11 | 32 | NO(2) | 48 hrs | 12.44 | 6 weeks |
| ¹ Numbers in parenthesis indicate the number of skin patches used. If after 1 hour the first patch had not caused contractions to stop, a second patch was applied. If after a further hour contractions had still not ceased the treatment was recorded as having failed. After failure of NO treatment salbutamol was administered. | | | | | |

Patients with significantly elevated CRH (1 and 10) delivered within the next few days inspite of treatment. Patients with lower levels of CRH did not deliver for a number of weeks.

The present inventor has shown that the timing of human parturition is influenced from an early stage in pregnancy by a longitudinal maturation process in the foetal-placental unit. The measurement of the maternal plasma CRH concentration (a placental trophoblast product) was utilised as a marker of this process. Plasma samples were obtained prospectively from 485 women during the second and third trimester and the relationship between maternal plasma CRH and the subsequent gestational age at the time of onset of labour was determined. The maternal plasma CRH concentration rose exponentially as pregnancy advanced (Figures 1 and 2), as previously described^{(5,6)}. Women delivering prematurely had elevated CRH levels from the earliest stage in pregnancy at which CRH can be measured by the assay used by the inventor. In this group the regression curve of plasma CRH versus gestational age was parallel to that observed in normal pregnancies but was significantly shifted to the left by a magnitude equivalent to the degree of prematurity at the time of delivery. Conversely, in women whose pregnancies continued beyond term the CRH curve was significantly shifted to the right with the magnitude of the shift again corresponding to the extent of post-maturity observed. Previous cross-sectional studies have reported elevated maternal plasma CRH levels at the time of preterm labour^{(7,8)}. The present inventor has now shown that the patterns of plasma CRH that are associated with aberrant timing of delivery are established by the end of the first trimester and persist throughout gestation. These data demonstrate the existence of a longitudinal process occurring in the placenta which influences the subsequent timing of delivery. This process may be analogous to a "placental clock" which triggers the onset of parturition at a predetermined time, and that the maternal plasma CRH level is an indicator of the rate of progress towards this event.

CRH may act directly as a trigger for the initiation of parturition. Any hypothesis about the action of CRH in pregnancy, however, must include consideration of the recently identified CRH-BP⁽⁴⁾. CRH-BP binds to the hormone in an equimolar ratio and prevents its recognition at the CRH receptor. It is known to be present in the maternal plasma during pregnancy⁽⁹⁾ but the relative concentrations of CRH and CRH-BP have not been reported and the bioactivity of placental CRH is therefore unknown. The CRH and CRH-BP concentrations were measured in duplicate aliquots of plasma obtained throughout pregnancy and parturition (Figure 3). CRH-BP was present greatly in excess of CRH until approximately 20 days before spontaneous term delivery. At this time the CRH-BP concentration fell while placental CRH secretion continued to rise exponentially, causing a rapid increase in the bioavailability of free circulating CRH in the final 3 weeks of gestation. There was no detectable difference in plasma CRH-BP levels between the groups of women delivering preterm, at term or post-term (data not shown). The fall in CRH-BP levels at term is likely to be a consequence of the rising CRH concentration since CRH has been shown to promote rapid clearance of CRH-BP from the circulation⁽¹⁰⁾.

There is a striking association between the timing of the increase in CRH bioavailability in late pregnancy and the onset of parturition. Although this does not prove that CRH directly initiates parturition, several lines of evidence suggest that this is likely. CRH receptors have been demonstrated in the myometrium⁽¹¹⁾ and foetal membranes⁽¹²⁾, and CRH has been shown to stimulate the release of prostaglandins from human decidua and amnion in-vitro⁽¹³⁾ and to potentiate the action of oxytocin in inducing myometrial contraction in-vitro⁽¹⁴⁾ and in-vivo⁽¹⁵⁾. CRH is also present in the amniotic fluid and the foetal circulation where it is capable of stimulating the foetal pituitary-adrenal axis to increase foetal adrenal glucocorticoid secretion, promoting foetal organ maturation⁽¹⁶⁾. Finally, prostaglandins and glucocorticoids induced by CRH in turn stimulate further placental CRH secretion⁽¹⁷⁻¹⁹⁾, creating positive feedback loops in the maternal, foetal and amniotic compartments to promote parturition and drive the onset of labour.

A critical event in the initiation of human parturition occurs when the maternal plasma CRH level, controlled by the "placental clock", rises to a level sufficient to fully saturate its binding-protein, thereby unmasking CRH bioactivity. This phenomenon is unique to the human species. In no other species does the placenta secrete large amounts of CRH near term and none other than humans have been shown to possess a circulating CRH-BP⁽²⁰⁾.

Many factors could affect the speed of the placental clock in an individual pregnancy; including genetic predisposition (some women experience repeated premature or post-mature deliveries), the rate of foetal organ maturation, and pathology in the mother or foetus. The trophoblast is strategically situated at the interface between the maternal and foetal circulations where its function can be influenced by humoral factors originating from either side. Placental CRH secretion is stimulated by inflammatory mediators, cytokines and stress hormones⁽¹⁹⁻²¹⁾ and inhibited by the nitric oxide (NO) donors⁽²²⁾. Since NO synthase has been demonstrated in the placenta⁽²³⁾, NO may participate in paracrine regulation of placental CRH expression.

The ability to determine at an early stage in pregnancy that the pregnancy may result in preterm or post-term delivery will allow the possibility to initiate medical management of the pregnancy so that a full term delivery may result. The present invention may also be useful to monitor the management of a high risk pregnancy.

It has recently been reported that the life expectancy of a given individual may be directly related to the speed of development of the foetus during gestation. Preterm delivery that results from the quicker development of a foetus during pregnancy may result in a shorter life expectancy of the person. Similarly, post-term delivery that results from the slower development of a foetus may result in a longer life expectancy of the person. As the growth of a foetus is affected by the level of CRH during pregnancy, the method of the present invention may also be used to indirectly determine the life expectancy of an individual at an early stage in pregnancy. High levels of CRH indicate the likelihood of premature delivery and thus resulting in a shorter life expectancy. Conversely, low levels of CRH indicate the likelihood of post-mature delivery and thus resulting in a longer life expectancy.

### INDUSTRIAL APPLICATION

The methods of the present invention have application in the management and care of pregnant patients.

### REFERENCES:

(1) In Williams' Obstetrics (eds Cunningham, F G, MacDonald P C, Gant N F, Leveno K J & Gilstrap L C) Vol 19th, 297-361 (Prentice-Hall International, 1993).
(2) Smith R, Cubis J, Brinsmead M W, et al. Psychosomatic Research 4(1), 53-69 (1990).
(3) Kleinbaum D G, Kupper L L and Muller K E in Applied regression analysis and other multivariable methods (eds Kleinbaum D G, Kupper L L and Muller K E) Vol 2nd, 263-271 (PWS-Kent, Boston, 1988).
(4) Potter E, Behan D P, Fischer W H, Linton E A, Lowry P J and Vale W W. Nature 349, 423-426 (1991).
(5) Campbell E A, Linton E A, Wolfe C D A, Scraggs P R, Jones M T and Lowry P J, J. Clin. Endocrinol. Metab. 64, 1054-1059 (1987).
(6) Sasaki A, Shinkawa O, Margioris A N, et al. J, Clin. Endocrinol. Metab. 64, 224-229 (1987).
(7) Wolfe C D A, Patel S P, Linton E A, et al. Br. J. Obstet. Gynecol. 95, 1003-1006 (1988).
(8) Kurki T, Laatikainen T, Salminen-Lappalainen K and Ylikorkala O. Br. J. Obstet. Gynecol. 98, 685-691 (1991).
(9) Linton E A, Perkins A V, Woods R J, et al. J. Clin. Endocrinol. Metab. 76, 260-262 (1993).
(10) Woods R J, Grossman A, Saphier P, et al. J. Clin. Endocrinol. Metab. 78, 73-76 (1994).
(11) Hillhouse E W, Grammatopoulos D, Milton N G N and Quartero H W P. J. Clin. Endocrinol. Metab. 76, 736-741 (1993).
(12) Petraglia F, Giardino M, Coukos G, Calza L, Vale W and Genazzani A R. Obstet. Gynecol. 75, 784-789 (1990).
(13) Jones S A and Challis J R G. Biochem. Biophys. Res. Commun. 159, 192-194 (1989).
(14) Quartero H W P & Fry C H. Placenta 10, 439-443 (1989).
(15) McLean M, Thompson D, Zhang H, Brinsmead M and Smith R. Eur. J Endocrinol 131, 167-172 (1994).
(16) Challis J R G and Hooper S. Bailliere's Clinical Endocrinology and Metabolism 3 No3, 781-793 (1989).
(17) Robinson B G, Emanuel R L, Frim D M & Majzoub J A. Proc. Natl. Acad. Sci. USA 85, 5244-5248 (1988).
(18) Jones S A, Brooks A N and Challis J R. J. Clin. Endocrinol. Metab. 68, 825-830 (1989).
(19) Petraglia F, Sutton S & Vale W. Am. J. Obstet. Gynecol. 160, 247-251 (1989).
(20) Smith R, Chan E C, Bowman M E, Harewood W J and Phippard A F. J. Clin. Endocrinol. Metab. 76, 1063-1068 (1993).
(21) Petraglia F, Garuti G C, De Ramundo B, Angioni S, Genazzani A R and Bilezikjian L M. Am. J. Obstet. Gynecol. 163, 1307-1312 (1990).
(22) Sun K, Smith R & Robinson P J. Basal and KC1 stimulated Corticotropin Releasing Hormone release from human syncytiotrophoblasts is inhibited by sodium nitroprusside. J. Clin. Endocrinol. Metab. in press 1994.
(23) Conrad K P, Vill M, McGuire P G, Dail W G and Davis A K. FASEB J. 7,(13):1269-1276 (1993).

## Claims

1. A method for detection or prediction of premature delivery in a pregnant human subject, the method comprising:
measuring during the first or second trimester of that pregnancy the maternal level of corticotrophin-releasing hormone (CRH);
comparing the level so obtained with the normal level of CRH, found in humans having a full term pregnancy and at the same stage of gestation as the pregnant human subject; and
determining that the delivery will occur sooner than full term if the level of CRH is elevated relative to the normal level of CRH.

2. A method as claimed in claim 1 wherein the pregnant human subject is experiencing preterm uterine contractions.

3. A method of early detection or prediction of post-mature delivery in a pregnant human subject comprising:
measuring during the first or second trimester of that pregnancy the maternal level of corticotrophin-releasing hormone (CRH);
comparing the level so obtained with the normal level of CRH found in humans having a full term pregnancy and at the same stage of gestation as the pregnant human subject; and
determining that the delivery will occur later than full term if the level of CRH is depressed relative to the normal level of CRH.

4. A method according to any one of claims 1 to 3 wherein the measuring of the CRH level is by an immunological method.

5. A method according to claim 4 wherein the immunological method is a radioimmunoassay.

## Patentansprüche

1. Verfahren zur Detektion oder Vorhersage einer Frühgeburt in einem schwangeren menschlichem Subjekt, wobei das Verfahren umfasst:
Messen des mütterlichen Niveaus an Corticotrophin-Releasing Hormon (CRH) während des ersten oder zweiten Trimesters der Schwangerschaft;
Vergleichen des so erhaltenen Niveaus mit dem normalen Niveau an CRH, das in Menschen vorgefunden wird, die eine Schwangerschaft von normaler Länge haben und im gleichen Stadium der Schwangerschaft wie das schwangere menschliche Subjekt sind; und
Bestimmen, dass die Geburt früher als zum normalen Zeitpunkt geschehen wird, wenn das Niveau an CRH relativ zum normalen Niveau an CRH erhöht ist.

2. Verfahren, wie in Anspruch 1 beansprucht, wobei im schwangeren menschlichen Subjekt verfrühte Gebärmutterkontraktionen auftreten.

3. Verfahren zur Detektion oder Vorhersage einer verspäteten Geburt in einem schwangeren menschlichem Subjekt, wobei das Verfahren umfasst:
Messen des mütterlichen Niveaus an Corticotrophin-Releasing Hormon (CRH) während des ersten oder zweiten Trimesters der Schwangerschaft;
Vergleichen des so erhaltenen Niveaus mit dem normalen Niveau an CRH, das in Menschen vorgefunden wird, die eine Schwangerschaft von normaler Länge haben und im gleichen Stadium der Schwangerschaft wie das schwangere menschliche Subjekt sind; und
Bestimmen, dass die Geburt später als zum normalen Zeitpunkt geschehen wird, wenn das Niveau an CRH relativ zum normalen Niveau an CRH vermindert ist.

4. Verfahren gemäß einem der Ansprüche 1 bis 3, wobei das Messen des CRH-Niveaus ein immunologisches Verfahren ist.

5. Verfahren gemäß Anspruch 4, wobei das immunologische Verfahren ein Radioimmunassay ist.

## Revendications

1. Méthode de détection et de prévision d'accouchement prématuré d'un sujet humain enceinte, la méthode comprenant :
- la mesure au cours du premier ou du deuxième trimestre de la grossesse du taux maternel de l'hormone corticolibérine (CRH);
- la comparaison du niveau ainsi obtenu avec le taux normal de CRH, trouvé chez une femme ayant une grossesse à terme et au même stade de la gestation que le sujet humain enceinte ; et
- la détermination que l'accouchement aura lieu avant terme si le taux de CRH est élevé par rapport au taux normal de CRH.

2. Méthode selon la revendication 1, pour laquelle le sujet humain enceinte ressent des contractions utérines avant terme.

3. Méthode de détection précoce et de prévision d'accouchement après terme d'un sujet humain enceinte, la méthode comprenant :
- la mesure au cours du premier ou du deuxième trimestre de la grossesse que du taux maternel de l' hormone corticolibérine (CRH);
- la comparaison du niveau ainsi obtenu avec le taux normal de CRH, trouvés chez une femme ayant une grossesse à terme et au même stade de la gestation que le sujet humain enceinte ; et
- la détermination que l'accouchement aura lieu après terme si le taux de CRH est inférieur par rapport au niveau normal de CRH.

4. Méthode selon l'une quelconque des revendications 1 à 3, pour laquelle la mesure du taux de CRH est effectuée par une méthode immunologique.

5. Méthode selon la revendication 4, pour laquelle la méthode immunologique est un dossage radio-immunologique.
